(19) Europäisches Patentamt
European Patent Office
Office européen des brevets

(11) Publication number: 0 496 452 A1

(12) **EUROPEAN PATENT APPLICATION**

(21) Application number: **92200111.0**

(22) Date of filing: **16.01.92**

(51) Int. Cl.$^5$: **C07K 11/02**, C12P 21/04, A61K 37/02

The applicant has filed a statement in accordance with Rule 28 (4) EPC (issue of a sample only to an expert). Accession number(s) of the deposit(s): ATCC 55140.

(30) Priority: **24.01.91 US 645535**

(43) Date of publication of application: **29.07.92 Bulletin 92/31**

(84) Designated Contracting States: **CH DE FR GB IT LI NL**

(71) Applicant: **MERCK & CO. INC.**
**126, East Lincoln Avenue P.O. Box 2000**
**Rahway New Jersey 07065-0900(US)**

(72) Inventor: **Lam, Yiu-Kuen Tony**
**25 Hamilton Lane North**
**Plainsboro, NJ 08536(US)**
Inventor: **Hensens, Otto D.**
**65 Alexander Drive**
**River Plaza, Red Bank, NJ 07701(US)**
Inventor: **Liesch, Jerrold M.**
**10 Sherbrooke Drive**
**Princeton Junction, NJ 08550(US)**
Inventor: **Zink, Deborah L.**
**37 Blenheim Road**
**Manalapan, NJ 07728(US)**
Inventor: **Huang, Leeyuan**
**33 Will Lane**
**Watchung, NJ 07060(US)**
Inventor: **Williams Jr., David L.**
**1545 Peach Tree Lane**
**Hatfield, PA 19440(US)**
Inventor: **Genilloud, Olga R.**
**Castello 107**
**E-28006 Madrid(ES)**

(74) Representative: **Thompson, John Dr. et al**
**Merck & Co., Inc. European Patent**
**Department Terlings Park Eastwick Road**
**Harlow, Essex CM20 2OR(GB)**

(54) **Novel endothelin receptor antagonists isolated from microbispora.**

(57) Novel cyclic depsipeptides derived from a culture of Microbispora are receptor antagonists of endothelin, a potent vasoconstrictor, and are thus useful in treating cardiovascular disorders. Endothelin has also other physiological effects, and the endothelin receptor antagonists of the present invention thus have also other therapeutic uses. The compounds are cyclic depsipeptides of N-(pyrrol-2-carboxy) - L-Phe, D-allo-Thr, D-Phe, D-Ala, D- or L-dihydroxyphenyl-Gly, and D-dihydroxyphenyl-Gly.

EP 0 496 452 A1

FIG. 1

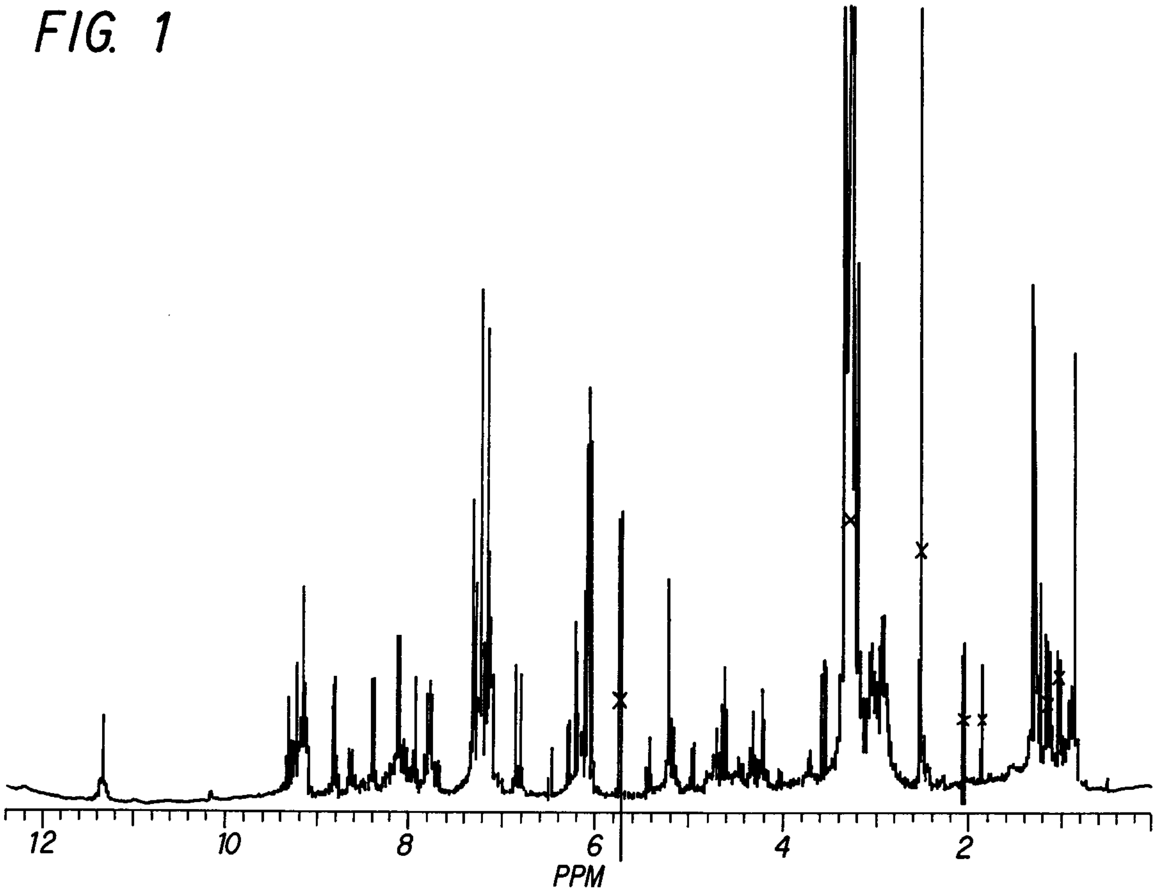

## BACKGROUND OF THE INVENTION

1. Field of the Invention

The present invention belongs to the field of receptor antagonists of endothelin. Endothelin (ET-1) itself is an endothelium-derived potent vasoconstrictor peptide consisting of 21 amino acids. The unusually prolonged vasoconstriction induced by endothelin in the presence or absence of extracellular $Ca^{2+}$ suggests that the action of this peptide may profoundly influence blood pressure regulation under normal and pathophysiological conditions. As described further below, endothelin also has a number of other physiological effects, and antagonists which bind to its receptors are thus expected to have advantageous pharmacological properties and corresponding therapeutic benefits.

2. Brief Description of the Prior Art

Endothelin was initially purified from the culture medium of porcine aortic endothelial cells; Yanagisawa et al. (1988) Nature 332, 411-415. Further investigations of the activity of endothelin have been described in publications such as the following:

- Takagi et al. (1988) Biochem. Biophys. Res. Commun. 157, 1164-1168.
- Sugiura et al. (1989) Biochem. Biophys. Res. Commun. 158, 170-176.
- Miller et al. (1989) J. Clin. Invest. 83, 317-320.
- Du Pont Biotech Update (1990).
- Warner et al. (1989) J. Cardiovasc. Pharmacol. 13(Suppl. 5), S85-S88.
- Yoshizawa et al. (1990) Science 247, 462-464.
- Bousso-Mittler et al. (1989) Biochem. Biophys. Res. Commun. 162, 952-957.
- Saito et al. (1989) Hypertension 14, 335-336.
- Tomita et al. (1989) New Engl. J. Med. 321, 1127.
- Kurihara et al. (1989) J. Cardiovasc. Pharmacol. 13(suppl. 5) S13-S17.
- Sugiura et al. (1989) Biochem. Biophys. Res. Commun. 161, 1220-1227.

U. S. Pat. No. 4,810,692 discloses only two immunosuppressant cyclodepsipeptides designated 55185 RP and 59451 RP, although they are represented by a more general formula which includes many stereoisomers not specified by the formula. While species 55185 RP and 59451 RP are characterized in the '692 patent by various chemical/physical data, they are, nevertheless, unspecfied stereoisomers that have been found to be different from cyclic depsipeptides I, II, and III of the present invention.

Thus, the present invention relates to the stereoisomers, compounds I, II, and III that, even though falling within the general formula of the '692 patent, are neither isolated nor suggested in said patent, and are produced by a different microorganism than that disclosed in the '692 patent. Moreover, compounds I, II, and III have strong endothelin receptor antagonist activity not disclosed for the species of the '692 patent, and on the basis of which they can be distinguished. Further, the two stereoisomers II and III of the present invention appear to have no significant immunosuppressant activity, although this may be attributable to assay differences.

## DESCRIPTION OF THE DRAWINGS

FIGS. 1, 2 and 3 of the accompanying drawings show respectively the $^1H$ NMR spectra for Compounds I, II and III, as described further below.

## SUMMARY OF THE INVENTION

The present invention relates to three novel cyclic depsipeptides (I, II, and III) active as endothelin receptor antagonists.

The present invention further relates to a method of preparing three novel cyclic depsipeptides (I, II and III) by culturing Microbispora sp. MA6857, ATCC 55140, and isolating the compounds from the fermentation broth.

The present invention also relates to the novel microorganism strain Microbispora sp. MA6857, ATCC 55140.

The present invention relates to a method of treating asthma, hypertension, renal failure particularly post-ischemic renal failure, cyclosporin nephrotoxicity, vasospasm, cerebral and cardiac ischemia, myocardial infarction, or endotoxin shock caused by or associated with endothelin (ET-1), comprising administering

to a patient in need of such treatment a therapeutically effective amount of one of three novel cyclopeptides (I, II or III).

The present invention further relates to a pharmaceutical composition for treating asthma, hypertension, renal failure particularly post-ischemic renal failure, cyclosporin nephrotoxicity, vasospasm, cerebral and cardiac ischemia, myocardial infarction, or endotoxin shock caused by or associated with endothelin (ET-1) comprising a therapeutically effective amount of one of three novel cyclopeptides (I, II or III) together with a pharmaceutically acceptable carrier therefor.

## DETAILED DESCRIPTION OF THE INVENTION

In accordance with the present invention there is provided an endothelin receptor antagonist cyclic depsipeptide of the formula:

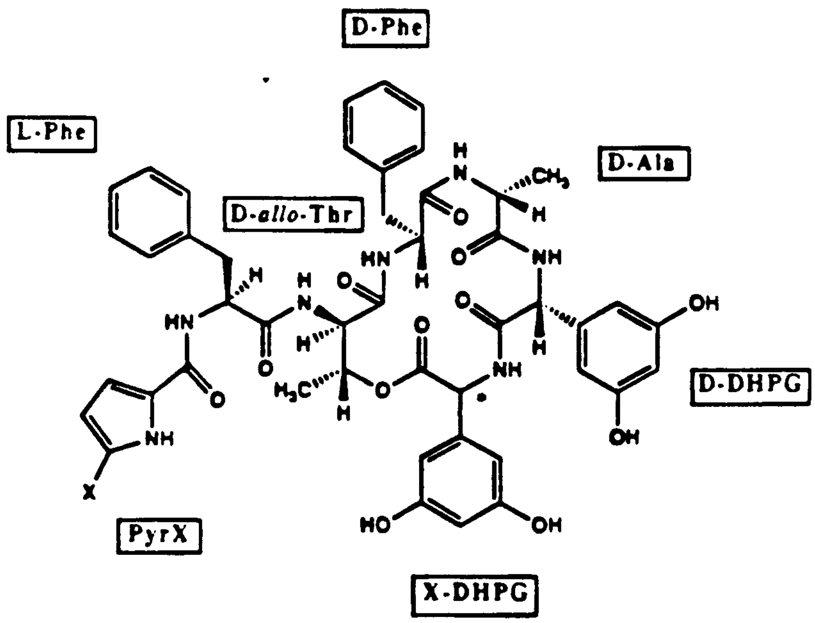

| | PyrX | X-DHPG |
|---|---|---|
| Component I: | X = H | D-DHPG |
| Component II: | X = Cl | L-DHPG |
| Component III: | X = Cl | D-DHPG |

## PROTON NUCLEAR MAGNETIC RESONANCE ($^1$H NMR)

The proton nuclear magnetic resonance ($^1H$ NMR) spectra for the three cyclic depsipeptides of the formula above are shown in FIGS. 1-3. The spectra were recorded at 400 MHz in DMSO-$d_6$ on a Varian XL400 NMR spectrometer. Chemical shifts are shown in ppm relative to tetramethylsilane (TMS) at zero ppm using the solvent peak at 2.49 ppm as internal standard.

For convenience, the data is also presented in tabular form immediately below.

TABLE I

$^1H$ NMR Assignments of Cyclic Depsipeptide I in DMSO-$d_6$ at 30° C Recorded at 400 MHz[a,b]

| Residue | $H_\alpha$ | $H_\beta$ | $H_\gamma$ | NH | Aromatic |
|---|---|---|---|---|---|
| D-Ala | 4.22 dq | 1.29 d (3H) | --- | 7.81 d | --- |
| | J = 7.5 | J = 7.5 | | J = 7.5 | |
| D-*allo*-Thr | 4.62 t | 5.18 dq | 0.84 d (3H) | 8.81 d | --- |
| | J ≅ 9.5 | J = 10, 6.5 | J = 6.5 | J = 9 | |
| L-Phe | 4.71 ddd | 3.06 dd | --- | 8.10d | 7.1-7.34 (10H) |
| | J = 5, 8.5, 10.5 | J = 5, 13.5 | | J = 8.5 | |
| | | 2.92 dd | | | |
| | | J = ~10.5, ~14 | | | |
| D-Phe | 4.33 dt | 2.99 dd | --- | 7.80 d | 7.1-7.34 (10H) |
| | J = 5.5, 8 | J = ~8, ~14 | | J = 7.5 | |
| | | 2.90 dd | | | |
| | | J = 5.5, ~14 | | | |
| D-DHPG | 5.22 d | --- | --- | 7.94 d | 6.07 d (2H)[c] |
| | J = 8 | | | J = 8 | J = 2 |
| | | | | | 6.11 t[c] |
| | | | | | J = 2 |
| D-DHPG | 5.22 d | --- | --- | 8.39 d | 6.02 d (2H)[c] |
| | J ≅ 10 | | | J = 9.5 | J = 2 |
| | | | | | 6.09 t[c] |
| | | | | | J = 2 |
| Pyrrole | --- | --- | --- | 11.32 br. m | 6.05 m |
| | | | | | 6.80 m |
| | | | | | 6.87 m |

[a,b,c] See after Table III.

TABLE II

$^1$H NMR Assignments of Cyclic Depsipeptide II in DMSO-$d_6$ at 30° C Recorded at 400 MHz[a,b]

| Residue | $H_\alpha$ | $H_\beta$ | $H_\gamma$ | NH | Aromatic |
|---|---|---|---|---|---|
| D-Ala | 3.94 dq<br>J = 5, 7.5 | 1.32 d (3H)<br>J = 7.5 | --- | 8.17 d<br>J = 5 | --- |
| D-*allo*-Thr | 4.45 t<br>J ≅ 9.5 | 4.96 dq<br>J = 10, 6.5 | 1.11 d (3H)<br>J = 6.5 | 8.60 d<br>J = ≅ 8 | --- |
| L-Phe | 4.79 ddd<br>J = 5, 9, 10 | 3.02 dd<br>J = 5, 13.5<br>2.88 dd<br>J = 10.5, 13.5 | --- | 8.12 d<br>J = 9 | 7.15 m (5H)[c] |
| D-Phe | 4.50 dt<br>J = 6, 9 | 3.04 dd<br>J = 6, 13.5<br>2.94 dd<br>J = 9, 13.5 | --- | 8.62 d<br>J = ≅8.5 | 7.29 m (2H)[c]<br>7.22 m (2H)[c]<br>7.04 m (1H)[c] |
| D-DHPG | 5.44 d<br>J = 9 | --- | --- | 7.78 d<br>J = 9 | 6.23 d (2H)<br>J = 2<br>6.15 t<br>J = 2 |
| D-DHPG | 5.25 d<br>J = 8 | --- | --- | 8.27 d<br>J = 8 | 6.17 d (2H)<br>J = 2<br>6.20 t<br>J = 2 |
| Cl-Pyrrole | --- | --- | --- | 12.16 br.s | 6.04 d<br>6.87 d<br>J = 4 |

TABLE III

$^1$H NMR Assignments of Cyclic Depsipeptide III in DMSO-$d_6$ at 30° C Recorded at 400 MHz[a,b]

| Residue | $H_\alpha$ | $H_\beta$ | $H_\gamma$ | NH | Aromatic |
|---|---|---|---|---|---|
| D-Ala | 4.22 dq<br>J = 7.5 | 1.31 d (3H)<br>J = 7.5 | --- | 7.81 d<br>J = 7.5 | --- |
| D-*allo*-Thr | 4.62 t<br>J ≅ 9.5 | 5.18 dq<br>J = 10, 6.5 | 0.85 d (3H)<br>J = 6.5 | 8.82 d<br>J = 9.5 | --- |
| L-Phe | 4.72 ddd<br>J = 4.5, 8.5, 10.5 | 3.07 dd<br>J = 4.5, 13.5<br>2.89 dd<br>J = 10.5, 13.5 | --- | 8.15 d<br>J = 8.5 | 7.1-7.33 (5H) |
| D-Phe | 4.33 dt<br>J = 5.5, 8 | 2.98 dd<br>J = 8, 13.5<br>2.89 dd<br>J = 5.5, 13.5 | --- | 7.79 d<br>J = 8 | 7.1-7.33 (10H) |
| D-DHPG | 5.23 d<br>J = 8 | --- | --- | 6.08 d<br>J = 8 | 6.08 d (2H)[c]<br>J = 2<br>6.12 t[c]<br>J = 2 |
| D-DHPG | 5.23 d<br>J ≅ 10 | --- | --- | 8.39 d<br>J = 10 | 6.03 d (2H)[c]<br>J = 2<br>6.10 t[c]<br>J = 2 |
| Cl-Pyrrole | --- | --- | --- | ~12.15 br.s | 6.87 d<br>6.02 d<br>J = 4 |

[a,b,c] See next page.

[a] Coupling constants ± 0.2 Hz.

[b] Except where otherwise noted, all chemical shift resonances correspond to one-proton intensity and are given in ppm using the solvent peak at δ 2.49 as interval standard.

[c] Assignments may be interchanged.

ABBREVIATIONS: In Tables I, II, and III above, the following abbreviations have been used: s = singlet; d = doublet; t = triplet; m = multiplet; br = broad

CARBON 13 NUCLEAR MAGNETIC RESONANCE ($^{13}C$ NMR)

In order to further characterize the cyclic depsipeptides II and III, $^{13}C$ NMR spectra were also recorded in DMSO-$d_6$ at 100 MHz on a Varian XL400 spectrometer at 30° C, with chemical shifts being given in ppm relative to tetramethylsilane (TMS) at zero ppm using the solvent peak at 39.5 ppm as internal standard. The spectral peaks are indicated below:

Compound II $^{13}C$ NMR Chemical Shifts:

16.6, 16.9, 36.8, 38.0, 51.8, 53.8, 54.9, 55.8, 56.16, 56.24, 70.6, 101.9, 102.2, 106.6 (2x), 106.8 (2x), 107.0, 111.8, 117.2, 125.7, 126.2, 126.3, 128.0 (2x), 128.1 (2x), 129.0 (2x), 129.2 (2x), 136.9, 137.8, 138.3, 139.6, 158.0 (2x), 158.2 (2x), 159.2, 168.4, 168.6, 168.9, 171.1, 171.5, 172.2 ppm.

Compound III $^{13}C$ NMR Chemical Shifts:

15.7, 17.7, 37.26, 37.31, 50.5, 54.2 (2x), 55.2, 56.75, 56.81, 69.0, 101.7, 102.2, 104.2 (2x), 104.7 (2x), 107.0, 111.7, 117.3, 125.6, 126.3, 126.4, 128.0 (2x), 128.3 (2x), 129.07 (2x), 129.12 (2x), 136.8, 137.8, 138.8, 139.4, 158.3 (4x), 159.5, 167.0, 168.4, 168.6, 170.4, 171.7, 172.0 ppm.

Stereochemistry

Cyclic depsipeptides I, II and III have the stereochemistries shown in the formula below, as determined by chromatographic and mass spectrometric comparison of the individual amino acids, obtained by partial or total hydrolysis, or of derivatives of these amino acids, with authentic reference standards:

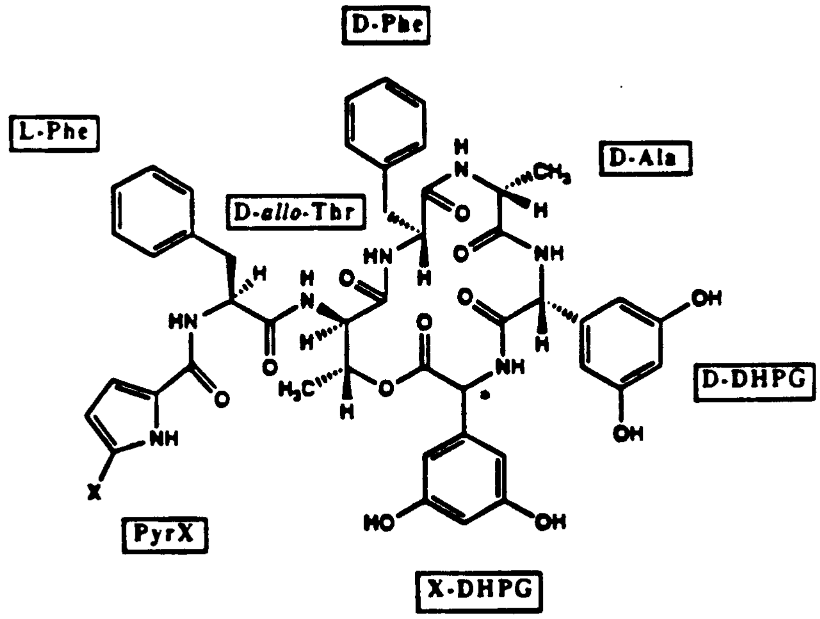

| | PyrX | X-DHPG |
|---|---|---|
| **Component I:** | **X = H** | **D-DHPG** |
| **Component II:** | **X = Cl** | **L-DHPG** |
| **Component III:** | **X = Cl** | **D-DHPG** |

Data obtained from $^{1}H/^{13}C$ NMR long range correlations and used for amino acid sequence determination of compounds I, II and III is illustrated in the formula below:

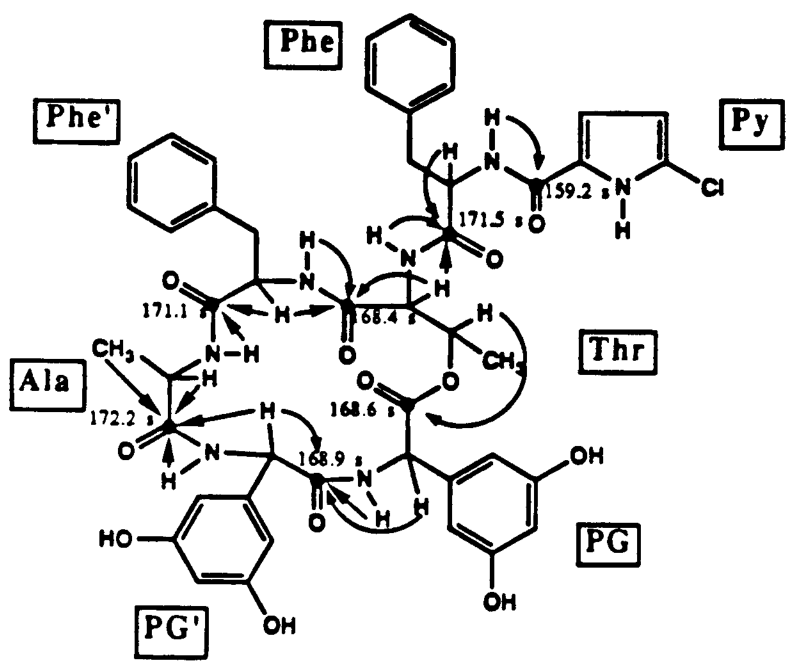

All three cyclopeptides I, II, and III have the same basic sequence and contain one equivalent each of D-allo-Thr, D-Ala, L-Phe, D-Phe, 5-chloropyrrole carboxylic acid (or pyrrole carboxylic acid in I), plus two equivalents of dihydroxyphenylglycine (DHPG). The Phe residues were differentiated on the basis of a

methanolysis product which contained only one of the Phe residues. The assignments of the DHPG residues were made using phenylglycine and tyrosine as model compounds. Both DHPG residues have the D configuration in the more active compounds I and III. Compound II contains both D and L DEPG and these residues have been differentiated in the sequence based on $^{1}H$ NMR data which exhibit a major variation in the chemical shift of the D-allo-Thr methyl resonance rather than the D-Ala methyl resonance.

Amino acid enantiomers were differentiated using the α-methylbenzylisothiocyanate (AMBI) method with standard reference amino acids except for DHPG. D-and L-Tyrosine and D- and L-phenylglycine were used as models for DHPG. In all cases, the L enantiomers eluted earlier than the corresponding D enantiomers. Approximate quantitation of the six peaks observed in each case is set out below in Table IV:

TABLE IV

| Approximate Quantitation of AMBI Derivatives | | | | | | |
|---|---|---|---|---|---|---|
| Compound | D-allo-Thr | D-Ala | L-DHPG | D-DHPG | L-Phe | D-Phe |
| I | 0.9 | *1.0 | 0.3 | 1.8 | 1.3 | 1.4 |
| II | 0.8 | 1.0 | 1.0 | 1.3 | 1.4 | 1.3 |
| III | 0.8 | 1.0 | 0.4 | 1.7 | 1.6 | 1.6 |

* Values standardized to D-Ala = 1.0.

The two peaks observed at approximately 12 and 13 minutes are assigned as the DHPG enantiomers. The compound I and III traces are quite similar and contain too equivalents of the 13 minute enantiomer. Component II on the other hand contains approximately 1 equivalent of each enantiomer. These traces are reproducible among a number of hydrolyzates from different isolations. The peaks observed at approximately 12 minutes in I and III result from partial racemization of the major, two-equivalent peak, at 13 minutes, during acid hydrolysis. GC-MS substantiated the assignment of allo-Thr since Thr and allo-Thr can be differentiated as their TMS derivatives based upon the ratios of their m/z 218/219 fragment ions. Treatment of compounds II and III with methanolic HCl afforded, among other components, the fragment allo-Thr--Phe--Ala--DHPG--DHPG(Me ester), whose sequence was verified by MS-MS. AMBI analyses of the acid hudrolyzates of these peptide fragments disclosed that both II and III contained D-Phe at the position between allo-Thr and Ala. These Phe residues are identical in I and III based on the similarity of their NMR spectra.

## Endothelin Activity and the Effects of Antagonizing Its Receptor Binding

Endothelin (ET-1), and two closely related bioactive peptides, ET-2 and ET-3, are widely distributed in mammalian tissues, and they can induce numerous biological responses in non-vascular as well as vascular tissues by binding to at least two distinct endothelin receptor subtypes. In addition to smooth muscle, neural and atrial sites, endothelin receptors may be found in gastrointestinal, urogenital, uteral and placental tissues.

Endothelin is a potent vasoconstrictor peptide and thus plays a role in vivo in arterial pressure-volume homeostasis. Not only peripheral, but coronary vascular resistance as well, is increased by endothelin. Cardiac output is decreased, while plasma renin activity is increased. There is a reduction in renal blood flow and glomerular filtration rate, while levels of atrial natriuretic factor, vasopressin, and aldosterone become elevated.

It is also considered, in accordance with the present invention, that antagonists for the endothelin receptor may be useful in preventing or reducing restenosis subsequent to denudation following angioplasty. Such denudation results from myointimal thickening following angioplasty, which is caused by increased endothelin release. Endothelin acts as a growth factor with resepct to smooth muscle and fibroblastic cells, and possibly other types of cells, as well.

Endothelin is also a neuropeptide, acting on the posterior pituitary, where it modulates the release of the neurosecretory hormones vasopressin and oxytocin. Endothelin released from the posterior pituitary also acts as a circulating hormone, having a wide range of actions as discussed further above. This includes effects on the endocrine system, especially the adrenal glands. Endothelin increases plasma levels of epinephrine.

Consequently, cyclic depsipeptides I, II, and III of the present invention, which are receptor antagonists of endothelin, have therapeutic usefulness in preventing, decreasing or modulating the various physiological

effects of endothelin discussed above, by wholly or partially blocking access of endothelin to its receptor.

Fermentation of Microbispora sp. MA6857

Cyclopeptides I, II and III of the present invention were isolated from a culture of a strain of Microbispora which is novel and which has been designated sp. MA6857. A sample of this microorganism was deposited under the Budapest Treaty at the American Type Culture Collection (ATCC), 12301 Parklawn Drive, Rockville, Maryland 20852, on January 17, 1991, where it has been assigned the accession number ATCC 55140. Based on the macro and micromorphology, the microorganism is a member of the genus Microbispora which is characterized by the formation of paired spores borne on an aerial mycelium. However, the microorganism has taxonomic and other features which distinguish it from the known strains of the genus Microbispora, and on that basis it is believed to be novel. It has, consequently, been designated herein Microbispora sp. MA6857.

The microorganism Microbispora sp. MA6857 was isolated from a culture prepared by well known methods from a soil sample originating in Cochin, India. In order to produce scaled-up culture batches for supplying additional quantities of cyclopeptides I, II and III, a seed culture was produced by inoculating 50 ml of aqueous nutrient medium, ATCC, in a 250 ml triple baffled erlenmeyer flask with 2 ml of refrigerated or thawed frozen vegatative mycelia. The nutrient medium ATCC has the following composition:

| ATCC | | |
|---|---|---|
| Glucose<br>Soluble starch<br>Yeast extract<br>N-Z amine E<br>$CaCO_3$<br>Beef extract<br>Bacto-peptone | 10.0g<br>20.0g<br>5.0g<br>5.0g<br>1.0g<br>3.og<br>5.0g | pH adjusted to 7.0 with NaOH prior to $CaCO_3$ addition |

The culture vessel was incubated at 28° C and shaken at 220 rpm for 96 hrs in order to obtain sufficient biomass for use as an inoculum for production medium. The production medium employed, SAM-4, contained sources of assimilable organic nutrients for growth of the culture in the form of dextrin, soybean flour and peptone. Its composition is set out below:

| SAM-4 | | |
|---|---|---|
| Dextrin<br>Soybean flour<br>Difco peptone<br>$CaCO_3$<br>Distilled $H_2O$ | 50.0g<br>30.0g<br>1.0g<br>5.0g<br>1000ml | pH adjusted to 7.0 with NaOH prior to $CaCO_3$ addition |

The production medium, 44 ml per 250 ml non-baffled erlenmeyer flask, was inoculated with 2 ml of seed culture per flask and incubated at 28° C and shaken at 220 rpm for 6 to 10 days.

From the seed culture described above, three different production media were prepared, as described below:

2E01 - 2 ml of cell suspension from a 4 day seed culture (obtained by inoculating ATCC medium, 50 ml per 250 ml baffled erlenmeyer, with thawed frozen mycelium and incubating at 28° C and 220 rpm) was used to inoculate 44 ml of SAM-4 production medium which was incubated for 10 days at 28° C and 220 rpm prior to harvest.

3G01 - 2 ml of cell suspension from a 4 day seed culture (obtained by inoculating ATCC medium with cells scraped from a BAM-2 agar slant) was used to inoculate 44 ml of SAM-4 production medium which was incubated for 6 days prior to harvest. Both seed and production media were incubated at 28° C and 220 rpm.

5L01 - 2 ml of cell suspension from a 4 day seed culture (obtained by inoculating ATCC medium with thawed frozen mycelia preserved in 10% glycerol) was used to inoculate 44 ml of SAM-4 production

medium which was incubated for 10 days prior to harvest. Both seed and production medium were incubated at 28° C and 220 rpm.

Isolation of Cyclopeptides I, II and III from Cultures 2E01 and 3G01

The 2E01 and 3G01 cultures were pooled (115 ml whole broth) and extracted with methyl ethyl ketone. The organic layer was flash evaporated under vacuum below 40° C on a roto-evaporator (dry weight = 89 mg) and was then dissolved in 0.2 ml of DMSO and chromatographed as one sample on a Whatman partisil 10 ODS-3 column (0.94 x 50 cm) at room temperature at 5 ml/min using a gradient of 0-100% acetonitrile in water (0-10 min, at 0%; 10-60 min, 0 to 100%, linear). Eighty one-minute fractions were collected, followed by a 200 ml acetonitrile wash. Fractions 38-44 were active in the $^{125}$I-ET-1 binding assay using bovine aortic tissue. Fractions 38-44 were then pooled, evaporated as above (dry weight = 16.6 mg), dissolved in 0.25 ml solution of methanol and dimethylsulfoxide (2:3) and rechromatographed on the same column, at room temperature. This time, a gradient of 20-75% methanol in water was employed (0-10 min, at 20%; 10-65 min, 20-75%, linear; 65-75 min, 75-100%, linear). Fractions 57-64 contained the activity and were pooled. Three active components were obtained by analytical HPLC analyses (Whatman partisil 5 ODS-3 0.46 x 10 cm; 50% aqueous methanol at a flow rate of 1ml/min at 40° C, and detection by monitoring UV adsorption at 215 nm) of these fractions: 58-59 (1.5 mg), 60-61 (4.5 mg), and 63-64 (1.6 mg). Further purification on E. Merck silica gel 60F TLC plates (10 x 20 cm, 0.2 mm thickness) using trifluoro acetic acid - methanol - dichloromethane, 1:5:95, revealed activity at the origin in each case. Subsequent purification of each of these active origin areas on Whatman partisil 10 ODS-3 (0.46 x 25 cm) at 40° C using isocratic elutions with 45%, 50% and 50% methanol/water at 1 ml/min for the respective fractions, collecting 1 minute fractions, resulted in activity in factions 13-17 (0.25 mg, component I), fractions 10-13 (3.2 mg, component II) and fractions 17-19 (0.64 mg, component III), as revealed by binding data and homogeneity indicated by analytical HPLC (Whatman partisil 5 ODS-3 0.46 x 10 cm; MeOH - $H_2O$, 45:55, at a flow rate of 1 ml/min at 40°C; and detection by monitoring UV absorption at 215 nm). UV, FT-IR, and $^1$H-NMR ($CD_3OD$) data showed the three components to be structurally related.

Isolation of Cyclopeptides I, II and III from Culture

Culture 5L01 (2700 ml whole broth, pH 8.4) was adjusted to pH 7.2 with dilute HCl and extracted with methyl ethyl ketone (3800 ml). The inactive aqueous layer was discarded. The active organic layer was flash evaporated under vacuum at below 40° C using a roto-evaporator (1.34 g dry weight; $EC_{50}$ = 25 µl WBE/ml) and chromatographed on a column of E. Merck silica gel 60 (50g, 40-63 µm) in $CH_2Cl_2$ with stepwise elution by $CH_2Cl_2$ (180 ml), 5% MeOH/$CH_2Cl_2$ (5 x 100 ml), 10% MeOH/$CH_2Cl_2$ (2 x 100 ml and 200 ml) and 200 ml each of 20, 30, 50 and 100% MeOH/$CH_2Cl_2$. HPLC analyses revealed desired components in factions 8 and 9 (i.e., the second and third 10% MeOH/$CH_2Cl_2$ eluates) which was confirmed by $^{125}$I-ET-1/bovine aorta binding data (91 and 74% inhibitions respectively at 900 µl WBE/ml). These fractions were pooled, flash evaporated under vacuum at below 40° C using a roto-evaporator (dry weight = 264 mg), and Chromatographed on a Whatman partisil 10 ODS-3 column (2.21 x 25 cm) at 40° C and a 15 ml/min flow rate using a 40 - 55 - 100% MeOH/$H_2O$ gradient elution (0-5 min, at 40%; 5-65 min, from 40-55%, linear; 65-120 min, from 55 to 100%, linear). One minute fractions were collected. Analytical HPLC (Whatman partisil 5 ODS-3 0.46 x 10 cm; MeOH - $H_2O$ 45:55, at a flow rate of 1 ml/min at 40° C; and detection by monitoring UV absorption at 215 nm) suggested pooling fractions 41-46, 51-55, and 64-72. Flash evaporation of these fractions gave 8.8 mg of compound I (fractions

TABLE V

| | Component I | Component II | Component III |
|---|---|---|---|
| $t_R$ (min)* | 4.75 | 6.08 | 9.40 |
| UV (MeOH): | | | |
| $\lambda_{max}$ nm (E%) | 209 (378.4) | 213 (483) | 212 (470.6) |
| | 270.5 (115) | 274.5 (218.8) | 273.5 (207.4) |
| $\lambda_{min}$ nm (E%) | 245 (53.9) | 246 (64) | 245.5 (72.2) |
| FT-IR: ** | | | |
| $\nu_{max}$ $cm^{-1}$ | 3287 | 3304 | 3304 |
| | 1733 | 1734 | 1733 |
| | 1668 | 1657 | 1662 |
| | 1563 | 1607 | 1558 |
| | 1521 | 1521 | 1519 |

* Analytical HPLC: column - Whatman partisil 5 ODS-3 0.46 x 10 cm; mobile phase - MeOH-$H_2O$, 45:55, @ 1 ml/min, 40° C; detection - UV 215 nm.

** Perkin-Elmer Model 1750 infrared fourier transform spectrophotometer; sample deposited on a zinc selenide crystal at ambient temperature.

Endothelin Receptor Binding Assay Results

The binding of cyclopeptides I, II and III to the endothelin receptor was determined in accordance with the assay described in detail immediately below. It is similar to the assay described in Ambar et al. (1989) Biochem. Biophys. Res. Commun. 158, 195-201; and Khoog et al. (1989) Febs Letters, 253, 199-202.

Endothelin-1 (ET-1) was purchased from Peptides International (Louisville, KY ). $^{125}$I-ET-1 (2000 Ci/mMol) was purchased from Amersham (Arlington Heights, IL).

Membranes were prepared from rat hippocampus, and rat or cow aorta. Dissected tissue was homogenized twice for 30 seconds with a Brinkman Polytron [setting 10, Generator PTA 20 TS (Westbury, NY)] in ice cold 250 mM sucrose, 50 mM Tris-HCl pH 7.4 with 7 µg/ml pepstatin A and 0.5 µg/ml leupeptin. The crude particulate matter was removed by centrifugation at 750 x g for 10 min. The membranes were sedimented from the supernatant fraction by centrifugation at 48,000 g for 30 min. Membrane pellets were resuspended in the above buffer with protease inhibitors. Aliquots of these suspensions were stored at -70° C.

Binding studies with $^{125}$I-ET-1 were conducted in 50 mM potassium phosphate pH 7.5 with 0.1% bovine serum albumin (BSA) using 12-well Shatron (Lier, Norway) cell harvester tube strips. $^{125}$I-ET-1 concentrations were 25 pM for hippocampus, 150 pM for aorta. Samples were dissolved in dimethylsulfoxide (DMSO). Upon addition of the sample, the final DMSO concentration was 3%. Membranes were added last to start the binding reaction. The reaction mixture was incubated at 37° C for 30 or 60 minutes. Binding reactions were terminated using a Skatron cell harvester by filtration through glass fiber filter pads presoaked with 2% BSA. The samples on the pads were immediately washed with 150 mM NaCl 0.1% BSA. The pads were punched out and radioactivity was evaluated in a Beckman Gamma 5500 gamma counter (Fullerton, CA). Nonspecific binding was determined in the presence of 100 nM ET-1. The results of this binding assay are set out in the table of values below:

TABLE VI

| Affinity for ET-A and ET-B Receptors | | |
|---|---|---|
| | $K_I$ (nM) vs. [$^{125}I$]-ET-1 | |
| | Rat Aorta (ET-A) | Rat Hippocampus (ET-B) |
| Compound I | 100 | 200 |
| Compound II | 3000 | 3000 |
| Compound III | 200 | 500 |

In accordance with the present invention, cyclic depsipeptides I, II and III of the present invention are useful in human therapy for treating asthma, hypertension, renal failure particularly post-ishemic renal failure, cyclosporin nephrotoxicity, vasospasm, cerebral and cardiac ischemia, myocardial infarction, or endotoxin shock caused by or associated with endothelin, comprising administering to a human patient in need of such treatment, a therapeutically effective amount of cyclic depsipeptides I, II or III.

The particular dosage to be administered in the course of such treatment is the result of a number of factors, such as the particular condition being treated, the route of administration, the age, sex, weight and general condition of the patient being treated, and whether acute or chronic treatment is envisioned. With those considerations in mind, it can be stated that, as a general matter, cyclic depsipeptides I, II and III of the present invention will be administered orally to a patient in dosage amounts between 1 and 200 mg/kg/day, and will be administered parenterally to a patient in dosage amounts between 0.5 and 100 mg/kg/day.

The present invention also relates to pharmaceutical compositions for treating asthma, hypertension, renal failure particularly post-ishemic renal failure, cyclosporin nephrotoxicity, vasospasm, cerebral and cardiac ischemia, myocardial infarction, or endotoxin shock caused by or associated with endothelin, comprising a therapeutically effective amount of cyclic depsipeptides I, II or III together with a pharmaceutically acceptable carrier therefor.

Where the pharmaceutical compositions of the present invention are for oral administration, e.g., as tablets, the active ingredient, i.e., cyclic depsipeptides I, II, or III, will be used in combination with other compounding ingredients such as talc, vegetable oils, polyols, benzyl alcohols, gums, gelatin, starches and other carriers, all of which are well known in the art. Where the pharmaceutical compositions of the present invention are for parenteral administration, the active ingredient will be dissolved or dispersed in a suitable liquid carrier, or emulsions may be formed using suitable emulsifying agents.

**Claims**

**1.** A cyclic depsipeptide receptor antagonist of andothelin of the formula:

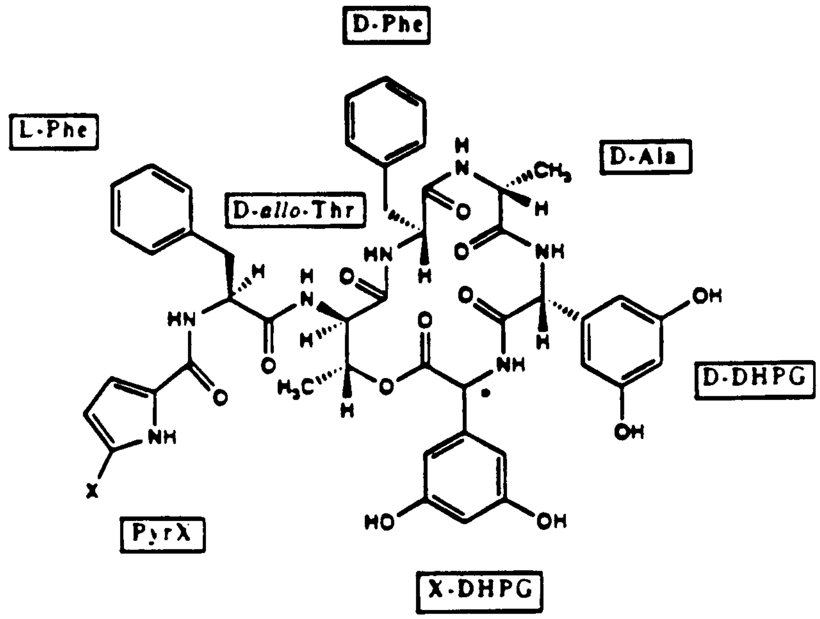

| | PyrX | X-DHPG |
|---|---|---|
| Component I: | X = H | D-DHPG |
| Component II: | X = Cl | L-DHPG |
| Component III: | X = Cl | D-DHPG |

2. A method of preparing a cyclic depsipeptide receptor antagonist of endothelin of Claim 1 comprising culturing Microbispora sp. MA6857, ATCC 55140, and isolating the desired compound from the fermentation broth.

3. The novel microorganism strain Microbispora sp. MA6857, ATCC 55140.

4. The use of a cyclic depsipeptide I, II or III of Claim 1 for the manufacture of a medicament for treating asthma, hypertension, renal failure, particularly post-ischemic renal failure, cyclosporin nephrotoxicity, vasospasm, cerebral and cardiac ischemia, myocardial infarction, or endotoxin shock caused by or associated with endothelin.

5. The use as claimed in Claim 4 wherein the medicament manufactured is adapted for the treatment of hypertension.

6. The use as claimed in Claim 4 wherein the medicament manufactured is adapted for the treatment of cyclosporin nephrotoxicity.

7. A pharmaceutical composition for treating asthma, hypertension, renal failure, particularly post-ischemic renal failure, cyclosporin nephrotoxicity, vasospasm, cerebral and cardiac ischemia, myocardial infarction, or endotoxin shock caused by or associated with endothelin, comprising a therapeutically effective amount of a cyclic depsipeptide I, II or III of Claim 1, together with a pharmaceutically acceptable carrier therefor.

FIG. 1

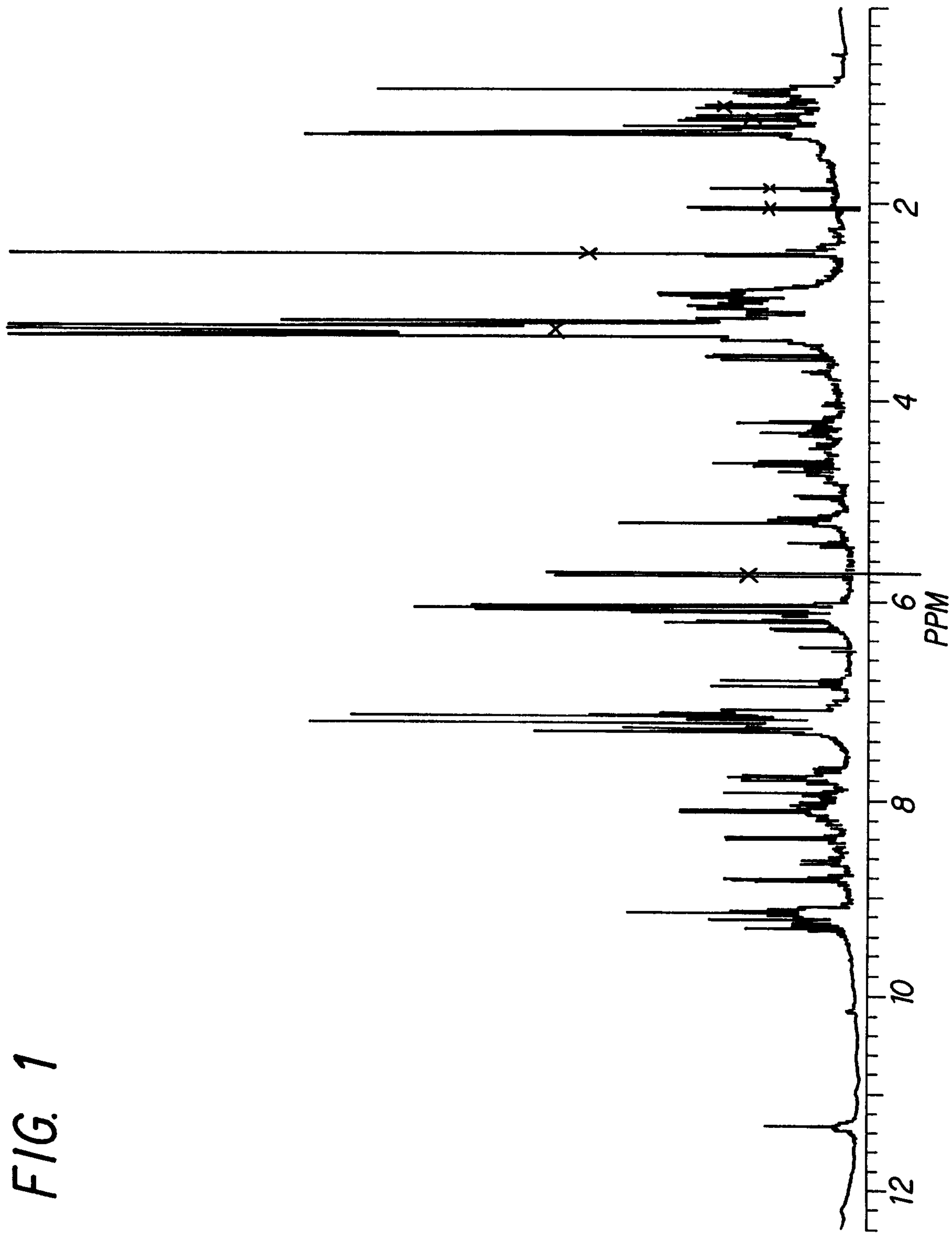

FIG. 2

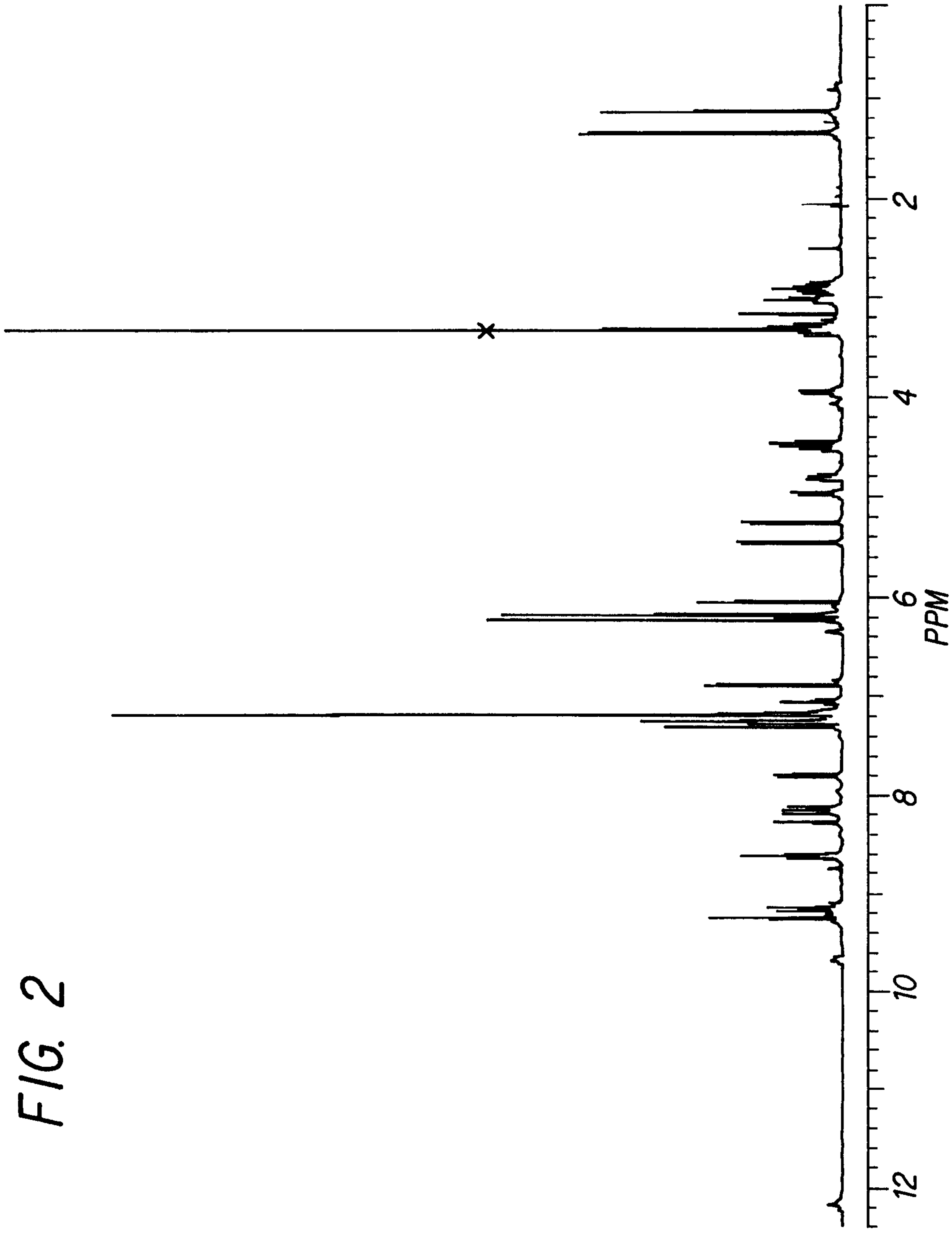

FIG. 3

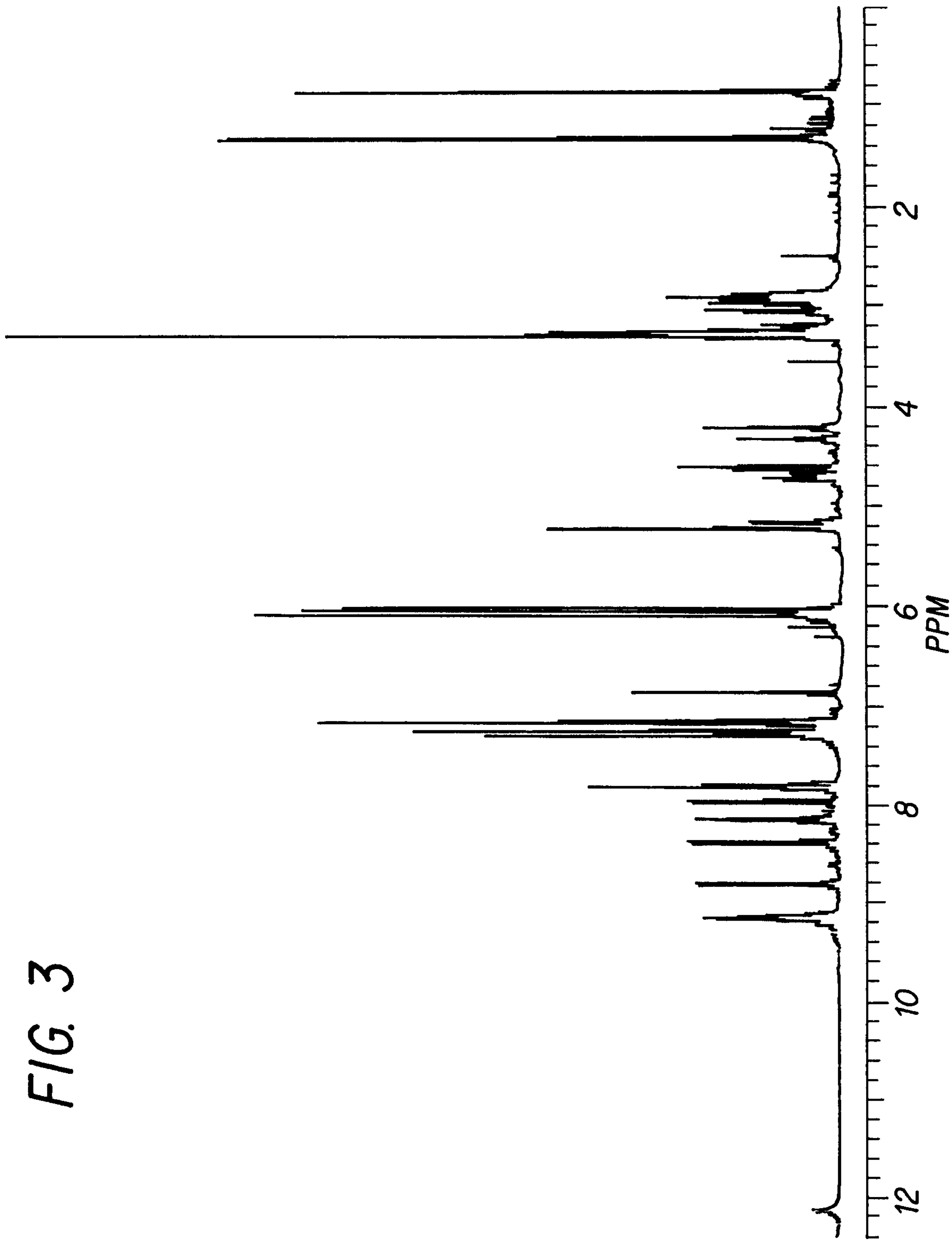

European Patent Office

# EUROPEAN SEARCH REPORT

Application Number: EP 92200111.0

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | EP - A - 0 246 975 (RHONE-POULENC SANTE) * Abstract * -- | 1-2 | C 07 K 11/02<br>C 12 P 21/04<br>A 61 K 37/02 |
| P,A | CHEMICAL ABSTRACTS, vol. 115, no. 19, November 11, 1991, Columbus, Ohio, USA IHARA, MASAKI et al. "An endothelin receptor antagonist isolated from Streptomyces misakiensis.", page 215, column 1, abstract-no. 199 551b & Biochem. Biophys. Res. Commun. 1991, 178(1), 132-7 ---- | 1,4-7 | |

TECHNICAL FIELDS SEARCHED (Int. Cl.5)

C 07 K 7/00
C 07 K 11/00
C 12 P 21/00

The present search report has been drawn up for all claims

| Place of search | Date of completion of the search | Examiner |
|---|---|---|
| VIENNA | 27-04-1992 | SCHARF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)